# EUROPEAN PATENT APPLICATION

(11) **EP 2 465 524 A1**
(43) Date of publication of application: **20.06.2012**
(21) Application number: 10804217.7
(22) Date of filing: 28.06.2010
(51) Int. Cl.: A61K 39/00, A61K 39/395, A61P 5/14, A61P 5/16, A61P 7/04, A61P 7/06, A61P 21/04, A61P 25/00, A61P 43/00, A61P 37/02, C07K 14/705, C07K 16/00, C07K 19/00

(54) **AUTOANTIBODY PRODUCTION INHIBITOR**

(30) Priority: 30.07.2009 JP 2009178120
(71) Applicant: Nihon Pharmaceuticals Co., Ltd., Tokyo 101-0031 (JP)
(72) Inventor: HOMMA, Masayuki, Narita-shi Chiba 286-0825 (JP)
(74) Representative: Patentanwälte Ruff, Wilhelm, Beier, Dauster & Partner
(86) International application number: PCT/JP2010/060973
(87) International publication number: WO 2011/013470

(57) **Abstract**

The present invention provides an autoantibody production inhibitor which can specifically suppress the autoantibodies and which can effectively prevent or treat the autoimmune disease of autoantibody type. According to the present invention, there is provided an autoantibody production inhibitor comprising, as an effective ingredient, a fusion protein which consists of a protein (X) containing a site recognized by autoantibodies which are a cause of the autoimmune disease of autoantibody type and a protein (A) containing a fragment which exhibits the antibody-dependent cellular cytotoxicity of the antibody heavy chain constant region.

SEQUENCE LISTING

<110>  Nihon Pharmaceutical Co., LTD.

<120>  Agent for inhibiting a production of autoantibody

<130>  F-3680PT

<150>  JP 2009-178120
<151>  2009-07-30

<160>  13

<170>  PatentIn version 3.1

<210>  1
<211>  232
<212>  PRT
<213>  homo sapiens

<400>  1

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Seite 1

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  2
<211>  232
<212>  PRT
<213>  homo sapiens

<400>  2

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  3
<211>  442
<212>  PRT
<213>  homo sapiens

<400>  3

Ser Glu His Glu Thr Arg Leu Val Ala Lys Leu Phe Lys Asp Tyr Ser
1               5                   10                  15

Ser Val Val Arg Pro Val Glu Asp His Arg Gln Val Val Glu Val Thr
            20                  25                  30

Val Gly Leu Gln Leu Ile Gln Leu Ile Asn Val Asp Glu Val Asn Gln
        35                  40                  45

Ile Val Thr Thr Asn Val Arg Leu Lys Gln Gln Trp Val Asp Tyr Asn
    50                  55                  60

Leu Lys Trp Asn Pro Asp Asp Tyr Gly Gly Val Lys Lys Ile His Ile
65                  70                  75                  80

Pro Ser Glu Lys Ile Trp Arg Pro Asp Leu Val Leu Tyr Asn Asn Ala
                85                  90                  95

Asp Gly Asp Phe Ala Ile Val Lys Phe Thr Lys Val Leu Leu Gln Tyr
            100                 105                 110

Thr Gly His Ile Thr Trp Thr Pro Pro Ala Ile Phe Lys Ser Tyr Cys
        115                 120                 125

Glu Ile Ile Val Thr His Phe Pro Phe Asp Glu Gln Asn Cys Ser Met
    130                 135                 140

Lys Leu Gly Thr Trp Thr Tyr Asp Gly Ser Val Val Ala Ile Asn Pro
145                 150                 155                 160

Glu Ser Asp Gln Pro Asp Leu Ser Asn Phe Met Glu Ser Gly Glu Trp
                165                 170                 175

Val Ile Lys Glu Ser Arg Gly Trp Lys His Ser Val Thr Tyr Ser Cys
            180                 185                 190

Cys Pro Asp Thr Pro Tyr Leu Asp Ile Thr Tyr His Phe Val Met Gln
        195                 200                 205

Arg Leu Glu Pro Lys Ser Cys Asp Lys Thr His Thr Cys Pro Pro Cys
    210                 215                 220

Pro Ala Pro Glu Leu Leu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Pro Ala Pro Ile Glu Lys Thr Ile Ser Lys Ala Lys Gly
                325                 330                 335

Gln Pro Arg Glu Pro Gln Val Tyr Thr Leu Pro Pro Ser Arg Asp Glu
            340                 345                 350

Leu Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  4
<211>  437
<212>  PRT
<213>  homo sapiens

<400>  4

Ser Glu His Glu Thr Arg Leu Val Ala Lys Leu Phe Lys Asp Tyr Ser
1               5                   10                  15

Ser Val Val Arg Pro Val Glu Asp His Arg Gln Val Val Glu Val Thr
            20                  25                  30

Val Gly Leu Gln Leu Ile Gln Leu Ile Asn Val Asp Glu Val Asn Gln
        35                  40                  45

Ile Val Thr Thr Asn Val Arg Leu Lys Gln Gln Trp Val Asp Tyr Asn
    50                  55                  60

Leu Lys Trp Asn Pro Asp Asp Tyr Gly Gly Val Lys Lys Ile His Ile
65                  70                  75                  80

Pro Ser Glu Lys Ile Trp Arg Pro Asp Leu Val Leu Tyr Asn Asn Ala
                85                  90                  95

Asp Gly Asp Phe Ala Ile Val Lys Phe Thr Lys Val Leu Leu Gln Tyr
            100                 105                 110

Thr Gly His Ile Thr Trp Thr Pro Pro Ala Ile Phe Lys Ser Tyr Cys
        115                 120                 125

Glu Ile Ile Val Thr His Phe Pro Phe Asp Glu Gln Asn Cys Ser Met
    130                 135                 140

Lys Leu Gly Thr Trp Thr Tyr Asp Gly Ser Val Val Ala Ile Asn Pro
145                 150                 155                 160

Glu Ser Asp Gln Pro Asp Leu Ser Asn Phe Met Glu Ser Gly Glu Trp
                165                 170                 175

Val Ile Lys Glu Ser Arg Gly Trp Lys His Ser Val Thr Tyr Ser Cys
            180                 185                 190

Cys Pro Asp Thr Pro Tyr Leu Asp Ile Thr Tyr His Phe Val Met Gln
        195                 200                 205

Arg Leu Pro Leu Tyr Phe Ile Val Asn Val Ile Ile Pro Cys Leu Leu
    210                 215                 220

Phe Ser Phe Leu Thr Gly Leu Val Phe Tyr Leu Pro Thr Asp Ser Gly
225                 230                 235                 240

Glu Lys Met Thr Leu Ser Ile Ser Val Leu Leu Ser Leu Thr Val Phe
                245                 250                 255

Leu Leu Val Ile Val Glu Leu Ile Pro Ser Thr Ser Ser Ala Val Pro

260                    265                    270

Leu Ile Gly Lys Tyr Met Leu Phe Thr Met Val Phe Val Ile Ala Ser
        275                 280                 285

Ile Ile Ile Thr Val Ile Val Ile Asn Thr His His Arg Ser Pro Ser
    290                 295                 300

Thr His Val Met Pro Asn Trp Val Arg Lys Val Phe Ile Asp Thr Ile
305                 310                 315                 320

Pro Asn Ile Met Phe Phe Ser Thr Met Lys Arg Pro Ser Arg Glu Lys
                325                 330                 335

Gln Asp Lys Lys Ile Phe Thr Glu Asp Ile Asp Ile Ser Asp Ile Ser
            340                 345                 350

Gly Lys Pro Gly Pro Pro Pro Met Gly Phe His Ser Pro Leu Ile Lys
        355                 360                 365

His Pro Glu Val Lys Ser Ala Ile Glu Gly Ile Lys Tyr Ile Ala Glu
    370                 375                 380

Thr Met Lys Ser Asp Gln Glu Ser Asn Asn Ala Ala Ala Glu Trp Lys
385                 390                 395                 400

Tyr Val Ala Met Val Met Asp His Ile Leu Leu Gly Val Phe Met Leu
                405                 410                 415

Val Cys Ile Ile Gly Thr Leu Ala Val Phe Ala Gly Arg Leu Ile Glu
            420                 425                 430

Leu Asn Gln Gln Gly
        435

<210>  5
<211>  462
<212>  PRT
<213>  homo sapiens

<400>  5

Ser Glu His Glu Thr Arg Leu Val Ala Lys Leu Phe Lys Asp Tyr Ser
1               5                   10                  15

Ser Val Val Arg Pro Val Glu Asp His Arg Gln Val Val Glu Val Thr
            20                  25                  30

Val Gly Leu Gln Leu Ile Gln Leu Ile Asn Val Asp Glu Val Asn Gln
        35                  40                  45

Ile Val Thr Thr Asn Val Arg Leu Lys Gln Gly Asp Met Val Asp Leu
    50                  55                  60

Pro Arg Pro Ser Cys Val Thr Leu Gly Val Pro Leu Phe Ser His Leu
65                  70                  75                  80

Gln Asn Glu Gln Trp Val Asp Tyr Asn Leu Lys Trp Asn Pro Asp Asp
                85                  90                  95

Tyr Gly Gly Val Lys Lys Ile His Ile Pro Ser Glu Lys Ile Trp Arg
            100                 105                 110

Pro Asp Leu Val Leu Tyr Asn Asn Ala Asp Gly Asp Phe Ala Ile Val
        115                 120                 125

Lys Phe Thr Lys Val Leu Leu Gln Tyr Thr Gly His Ile Thr Trp Thr
    130                 135                 140

Pro Pro Ala Ile Phe Lys Ser Tyr Cys Glu Ile Ile Val Thr His Phe
145                 150                 155                 160

Pro Phe Asp Glu Gln Asn Cys Ser Met Lys Leu Gly Thr Trp Thr Tyr
                165                 170                 175

Asp Gly Ser Val Val Ala Ile Asn Pro Glu Ser Asp Gln Pro Asp Leu
            180                 185                 190

Ser Asn Phe Met Glu Ser Gly Glu Trp Val Ile Lys Glu Ser Arg Gly
        195                 200                 205

Trp Lys His Ser Val Thr Tyr Ser Cys Cys Pro Asp Thr Pro Tyr Leu
    210                 215                 220

Asp Ile Thr Tyr His Phe Val Met Gln Arg Leu Pro Leu Tyr Phe Ile
225                 230                 235                 240

Val Asn Val Ile Ile Pro Cys Leu Leu Phe Ser Phe Leu Thr Gly Leu
                245                 250                 255

Val Phe Tyr Leu Pro Thr Asp Ser Gly Glu Lys Met Thr Leu Ser Ile
            260                 265                 270

Ser Val Leu Leu Ser Leu Thr Val Phe Leu Leu Val Ile Val Glu Leu
        275                 280                 285

Ile Pro Ser Thr Ser Ser Ala Val Pro Leu Ile Gly Lys Tyr Met Leu
    290                 295                 300

Phe Thr Met Val Phe Val Ile Ala Ser Ile Ile Ile Thr Val Ile Val
305                 310                 315                 320

Ile Asn Thr His His Arg Ser Pro Ser Thr His Val Met Pro Asn Trp
                325                 330                 335

Val Arg Lys Val Phe Ile Asp Thr Ile Pro Asn Ile Met Phe Phe Ser
            340                 345                 350

Thr Met Lys Arg Pro Ser Arg Glu Lys Gln Asp Lys Lys Ile Phe Thr
        355                 360                 365

Glu Asp Ile Asp Ile Ser Asp Ile Ser Gly Lys Pro Gly Pro Pro Pro
    370                 375                 380

Met Gly Phe His Ser Pro Leu Ile Lys His Pro Glu Val Lys Ser Ala
385                 390                 395                 400

Ile Glu Gly Ile Lys Tyr Ile Ala Glu Thr Met Lys Ser Asp Gln Glu
                405                 410                 415

Ser Asn Asn Ala Ala Ala Glu Trp Lys Tyr Val Ala Met Val Met Asp
            420                 425                 430

His Ile Leu Leu Gly Val Phe Met Leu Val Cys Ile Ile Gly Thr Leu
        435                 440                 445

Ala Val Phe Ala Gly Arg Leu Ile Glu Leu Asn Gln Gln Gly
    450                 455                 460


<210>  6
<211>  210
<212>  PRT
<213>  homo sapiens

<400>  6

Ser Glu His Glu Thr Arg Leu Val Ala Lys Leu Phe Lys Asp Tyr Ser
1               5                   10                  15

Ser Val Val Arg Pro Val Glu Asp His Arg Gln Val Val Glu Val Thr
            20                  25                  30

Val Gly Leu Gln Leu Ile Gln Leu Ile Asn Val Asp Glu Val Asn Gln
        35                  40                  45

Ile Val Thr Thr Asn Val Arg Leu Lys Gln Gln Trp Val Asp Tyr Asn
    50                  55                  60

Leu Lys Trp Asn Pro Asp Asp Tyr Gly Gly Val Lys Lys Ile His Ile
65                  70                  75                  80

Pro Ser Glu Lys Ile Trp Arg Pro Asp Leu Val Leu Tyr Asn Asn Ala
                85                  90                  95

Asp Gly Asp Phe Ala Ile Val Lys Phe Thr Lys Val Leu Leu Gln Tyr
            100                 105                 110

Thr Gly His Ile Thr Trp Thr Pro Pro Ala Ile Phe Lys Ser Tyr Cys
        115                 120                 125

Glu Ile Ile Val Thr His Phe Pro Phe Asp Glu Gln Asn Cys Ser Met
    130                 135                 140

Lys Leu Gly Thr Trp Thr Tyr Asp Gly Ser Val Val Ala Ile Asn Pro
145                 150                 155                 160

Glu Ser Asp Gln Pro Asp Leu Ser Asn Phe Met Glu Ser Gly Glu Trp
                165                 170                 175

Val Ile Lys Glu Ser Arg Gly Trp Lys His Ser Val Thr Tyr Ser Cys
            180                 185                 190

Cys Pro Asp Thr Pro Tyr Leu Asp Ile Thr Tyr His Phe Val Met Gln
        195                 200                 205

Arg Leu
    210

<210>  7
<211>  442
<212>  PRT
<213>  homo sapiens

<400>  7

Ser Glu His Glu Thr Arg Leu Val Ala Lys Leu Phe Lys Asp Tyr Ser
1               5                   10                  15

Ser Val Val Arg Pro Val Glu Asp His Arg Gln Val Val Glu Val Thr
            20                  25                  30

Val Gly Leu Gln Leu Ile Gln Leu Ile Asn Val Asp Glu Val Asn Gln
        35                  40                  45

Ile Val Thr Thr Asn Val Arg Leu Lys Gln Gln Trp Val Asp Tyr Asn
    50                  55                  60

Leu Lys Trp Asn Pro Asp Asp Tyr Gly Gly Val Lys Lys Ile His Ile
65                  70                  75                  80

Pro Ser Glu Lys Ile Trp Arg Pro Asp Leu Val Leu Tyr Asn Asn Ala
                85                  90                  95

Asp Gly Asp Phe Ala Ile Val Lys Phe Thr Lys Val Leu Leu Gln Tyr
            100                 105                 110

Thr Gly His Ile Thr Trp Thr Pro Pro Ala Ile Phe Lys Ser Tyr Cys
        115                 120                 125

Glu Ile Ile Val Thr His Phe Pro Phe Asp Glu Gln Asn Cys Ser Met
    130                 135                 140

Lys Leu Gly Thr Trp Thr Tyr Asp Gly Ser Val Val Ala Ile Asn Pro
145                 150                 155                 160

Glu Ser Asp Gln Pro Asp Leu Ser Asn Phe Met Glu Ser Gly Glu Trp
                165                 170                 175

Val Ile Lys Glu Ser Arg Gly Trp Lys His Ser Val Thr Tyr Ser Cys
            180                 185                 190

Cys Pro Asp Thr Pro Tyr Leu Asp Ile Thr Tyr His Phe Val Met Gln
        195                 200                 205

Arg Leu Glu Pro Lys Ser Cys Asp Lys Thr His Thr Cys Pro Pro Cys
    210                 215                 220

Pro Ala Pro Glu Leu Leu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Pro Ala Pro Ile Glu Lys Thr Ile Ser Lys Ala Lys Gly
                325                 330                 335

Gln Pro Arg Glu Pro Gln Val Tyr Thr Leu Pro Pro Ser Arg Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  8
<211>  39
<212>  DNA
<213>  Artificial

<220>
<223>  primer

<400>  8
aagctagcgc cgccaccatg aaacacctgt ggttcttcc                              39

<210>  9
<211>  26
<212>  DNA
<213>  Artificial

<220>
<223>  primer

<400>  9
aagaattcgg acaggaccca tctggg                                            26

<210>  10
<211>  39
<212>  DNA
<213>  Artificial

<220>
<223>  primer

<400>  10
agatgggtcc tgtcctccga acatgagacc cgtctggtg                              39

<210>  11
<211>  45
<212>  DNA
<213>  Artificial

<220>
<223>  primer

<400>  11
gttttgtcac aagatttggg ctccaggcgc tgcatgacga agtgg                       45

<210>  12
<211>  45
<212>  DNA
<213>  Artificial

<220>
<223>  primer

<400>  12
acttcgtcat gcagcgcctg gagcccaaat cttgtgacaa aactc                45

<210>  13
<211>  30
<212>  DNA
<213>  Artificial

<220>
<223>  primer

<400>  13
aagtttaaac tcatttaccc ggagacaggg                                 30

## Description

### Technical Field of the Invention

The present invention relates to an autoantibody production inhibitor which can effectively prevent and treat an autoimmune disease of autoantibody type such as myasthenia gravis by neutralizing autoantibodies and inhibiting the autoantibody production.

### Background Art

Immune system inherently has a role of recognizing and eliminating a foreign body such as bacterium or virus which is different from the self but, sometimes, it excessively reacts with one's own normal cells and tissues and attacks them. Autoimmune disease is a general name for the diseases resulted by such a state. Among them, a disease caused by the reaction of autoantibodies (antibodies which recognize one's own cells and tissues as an antigen) with autoantigen (one's own cells and tissues) is called autoimmune disease of autoantibody type. Examples of the autoimmune disease of autoantibody type include myasthenia gravis, hemolytic anemia of autoimmune type, idiopathic thrombocytopenic purpura, neutropenia of autoimmune type, hyperthyroidism caused by anti-TSH antibody, acute encephalitis of autoantibody type, Hashimoto encephalopathy and non-herpetic marginal encephalitis.

As to a treating method for autoimmune disease of autoantibody type, administration of steroidal agents or immunosuppressants has been conducted already but, since any of those drugs does not specifically suppress the autoantibodies but generally suppress the immunoreaction, the drugs have no specificity and the methods are not a sufficiently effective treating method.

With regard to myasthenia gravis which is one of the representative examples of the autoimmune diseases, there is no already-known treating agent for the fundamental treatment therefor as well but merely the above steroidal agents and immunosuppressants and/or cholinesterase inhibitors have been mostly used (Non-Patent Document 1). Particularly, for the use of the cholinesterase inhibitors, there is a problem that establishment of the dose thereof is difficult. Although a plasma exchange therapy is also applied, a cost of as high as not less than one million yen is needed for one treatment while a subsidy for the medical expenses of myasthenia gravis according to the system for Diagnosis Procedure Combination is only about six hundred thousand yen whereby the burden at the medical care site is big. Further, there is a problem that duration of the effect of the plasma exchange therapy is as short as only about one month. Although thymectomy is also used and exhibits an effect to some extent, there is a problem of uneasiness of patients for the excision or a problem of the cost and there is further problem that the thymectomy cannot be applied to small children whose immune mechanism has not been developed yet and to patients of immunodeficiency.

As a treating method for myasthenia gravis, effectiveness of gamma-globulin preparations have been confirmed in recent years and some pharmaceutical manufacturers are now conducting clinical tests therefor (Non-Patent Document 2). However, since gamma-globulin preparations are biological preparations derived from human plasma, there may be a risk of infection due to unknown virus, etc. In addition, dose of the gamma-globulin preparations is high and, even if actually materialized, it is expected that burdens in terms of cost and time for patients and medical care sites will be considerably high.

### Prior Art Documents

### Non-Patent Documents

Non-Patent Document 1: Trends of clinical test studies for myasthenia gravis, Nippon Rinsho, Vol. 66, No. 6, pp. 1155-1157
Non-Patent Document 2: High-dose therapy by immunoglobulin, Shinkei Chiryo, Vol. 25, No. 6, pp. 689-692

### Disclosure of the Invention

### Problem that the Invention is to Solve

The present invention has been created in view of the current status of the prior art as such and an object of the present invention is to provide an autoantibody production inhibitor which can specifically suppress the autoantibodies and which can effectively prevent or treat the autoimmune disease of autoantibody type.

### Means for Solving the Problem

As a method for the treatment of autoimmune disease of autoantibody type, the present inventor firstly thought of an idea that an antibody which reacts only with autoantibodies is prepared in a recombinant protein and the resulting antibody is administered to a patient whereby the autoantibodies in the body of the patient bind to this antibody and are decomposed. However, since the autoantibodies of the autoimmune disease of autoantibody type are not one specific antibody but are composed of a group of various antibodies, it is very difficult to prepare an anti-idiotypic antibody which reacts with all of the antibodies as such whereby it has been believed that possibility of realization of such a method is low.

Under such circumstances, the present inventor gave up to use an antibody reacting with autoantibodies as a treating method for autoimmune disease of autoantibody type. Instead, he found that, by preparing an autoantigen which is recognized by autoantibodies in a recombinant protein and administering this artificial autoantigen to a patient as a decoy, the autoantibodies in the body of the patient bind to this artificial autoantigen, and the autoantibodies are neutralized so that the autoantibodies cannot react with the autoantigen in the patient himself any longer. It has been further found that, when this artificial autoantigen is fused with constant region of antibody heavy chain, production of the autoantibodies can also be specifically suppressed whereupon the present invention has now been achieved.

Thus, according to the present invention, there is provided an autoantibody production inhibitor comprising, as an effective ingredient, a fusion protein which consists of a protein (X) containing a site recognized by autoantibodies which are a cause of the autoimmune disease of autoantibody type and a protein (A) containing a fragment which exhibits the antibody-dependent cellular cytotoxicity of the antibody heavy chain constant region.

The preferred embodiments of the autoantibody production inhibitor according to the present invention comprise the following (1) to (9).
(1) The protein (A) contains a Fc region of the antibody heavy chain, antibody heavy chain CH1 region or a part thereof.
(2) The protein (A) consists of amino acid sequence of SEQ ID No: 1 or SEQ ID No: 2.
(3) The protein (X) is a nicotinic acetylcholine receptor α1 (nAChRα1) subunit or a part thereof.
(4) The protein (X) is isoform 1 and/or isoform 2 of nAChRα1 subunit or a part thereof.
(5) The isoform 1 consists of amino acid sequence of SEQ ID No: 4.
(6) The isoform 2 consists of amino acid sequence of SEQ ID No: 5.
(7) The protein (X) consists of amino acid sequence of N-terminal extracellular region of isoform 1 and/or isoform 2 of nAChRα1 subunit, or consists of amino acid sequence wherein one or several amino acid(s) is/are deleted, added and/or substituted in said amino acid sequence.
(8) The fusion protein consists of amino acid sequence of SEQ ID No: 3, or consists of amino acid sequence wherein one or several amino acid (s) is/are deleted, added and/or substituted in the amino acid sequence of SEQ ID No: 3.
(9) The autoantibody production inhibitor is in a form of injection and suppository.

### Advantages of the Invention

The autoantibody production inhibitor of the present invention neutralizes the autoantibodies existing in the body of a patient suffering from autoimmune disease of autoantibody type and also inhibits the autoantibody production whereby it can specifically suppress the autoantibodies. Accordingly, when the autoantibody production inhibitor of the present invention is used, it is now possible to effectively prevent and treat various autoimmune diseases of autoantibody type such as myasthenia gravis.

### Brief Description of the Drawings

Fig. 1 is a schematic chart of vector pcDNA3.1-AChR-Fc for expressing a fusion protein (α1-Fc) prepared in Examples.
Fig. 2 shows a silver-stained image (left) and a western blotting image (right) of purified fusion protein (α1-Fc) in non-reduced and reduced states after SDS-PAGE.
Fig. 3 shows a binding of a fusion protein α1-Fc to a hybridoma Mab35. Concerning the adding concentration (µg/ml) of the fusion protein α1-Fc, A is no addition, B is 0.1, C is 0.36, D is 1, E is 3.6, F is 10, G is 36 and H is 100.
Fig. 4 shows an ADCC activity of a fusion protein α1-Fc to a hybridoma Mab35. A horizontal axis shows the ratio of NK 92 which is an effector cell to a hybridoma Mab35 which is a target cell while a vertical axis shows cellular cytotoxicity (%). ◆ shows cellular cytotoxicity when no antibody is added, ■ shows cellular cytotoxicity when a fusion protein α1-Fc is added, ▲ shows cellular cytotoxicity when Avastin is added and × shows cellular cytotoxicity when Enbrel is added. • shows total cell numbers.

### Best Mode for Carrying Out the Invention

The fusion protein which is an effective ingredient of the autoantibody production inhibitor of the present invention is a fusion product of a protein (X) containing a site recognized by autoantibodies which are a cause of the autoimmune disease of autoantibody type with a protein (A) containing a fragment which exhibits the antibody-dependent cellular cytotoxicity of the antibody heavy chain constant region. The protein (X) corresponds to an autoantigen to autoantibodies or a part thereof and plays a role of decoy binding to autoantibodies as a substitute for the autoantigen of a patient. Thus, when the fusion protein of the present invention is administered to a patient suffering from autoimmune disease of autoantibody type, the autoantibodies in the body of the patient recognize the protein (X) part in the fusion protein as autoantigen and binds to this part. Since the bound autoantibodies cannot bind to the autoantigen which is inherently present in the body of the patient any longer, the autoantibodies can be neutralized by this method and generation of symptom of the autoimmune disease by binding of the autoantibodies to the autoantigen of the patient can be suppressed. Although the autoantibodies are not one specific antibody but are composed of a group of various antibodies, any of the antibodies is common in such a view that it has a function of recognizing the autoantigen. Accordingly, when the fusion protein of the present invention which acts as a decoy of autoantigen is used, one fusion protein can neutralize a group of various antibodies, and thus there is no need to separately prepare each fusion protein for each of various antibodies.

The fusion protein of the present invention contains a protein (A) which contains a fragment exhibiting the antibody-dependent cellular cytotoxicity of the antibody heavy chain constant region in addition to a protein (X) which acts as a decoy for an autoantigen. This protein (A) also plays a role of exhibiting the antibody-dependent cellular cytotoxicity (ADCC activity). Autoantibodies are produced by B cells in the blood. Antibodies of cell surface presenting type having the same antigen binding site as the autoantibodies exist on the surface of the B cells as a B cell receptor. Accordingly, when the fusion protein of the present invention is administered to a patient, some of the protein binds to the autoantibodies in the body of the patient as mentioned above while some other binds to the antibodies on the surface of B cells (B cell receptor) which produce the autoantibodies. When the fusion protein of the present invention binds to the B cell receptor, effector cells such as NK cell bind to a protein (A) part in the fusion protein via an Fc receptor of the effector cell, exhibit the antibody-dependent cellular cytotoxicity (ADCC activity) and attack the B cells binding to the fusion protein to kill them. As such, in accordance with the present invention, the outcome is not only that the autoantibodies existing in the body are neutralized to prevent the binding of the autoantibodies to the autoantigen but also that the specific B cells which are a production source of the autoantibodies can be selectively killed. Accordingly, the fusion protein of the present invention can prevent or treat the autoimmune disease of autoantibody type by two ways which are inhibition of the autoantibody production and neutralization of the produced autoantibodies.
Incidentally, as an idea of using a decoy in a way similar to the fusion protein of the present invention, Japanese Patent Application Laid-Open (JP-A) No. 502538/90 by Jonathan M. Gershoni discloses a molecular decoyant. This molecular decoyant corresponds to the minimum partial fragment of natural receptor to foreign body showing undesirable action. The idea of Gershoni is to suppress the binding of the exogenous antigen to a living body receptor by a competitive action between exogenous antigen and decoyant. In this publication, the above foreign body is an exogenous antigen such as virus, bacterium and toxin and there is no disclosure at all for autoantibodies. Further, the molecular decoyant in this publication is used solely and is not bound to an antibody heavy chain constant region.
Further, as an idea of killing the B cells, Japanese Patent Application Laid-Open (JP-A) No. 2008-515926 by Anand Iyer et al. discloses that B cells are killed by administering, to a patient suffering from autoimmune disease, a composite where a cytotoxic substance is added to an antibody to a B cell surface antigen. Although this composite can kill the B cells, it cannot neutralize the produced autoantibodies. It is mentioned that, for the autoantibodies, an anti-cytokine preparation needs to be administered separately.

The protein (X) in the fusion protein of the present invention corresponds to an autoantigen, or a part thereof, to autoantibodies which are a cause of autoimmune disease of autoantibody type in the patient to be prevented or treated and is decided depending upon the autoimmune disease in the patient to be prevented or treated. For example, in the case of prevention and treatment of myasthenia gravis, since myasthenia gravis is a disease resulted by such a cause that anti-nicotinic acetylcholine receptor antibodies (autoantibodies) bind to a nicotinic acetylcholine receptor (autoantigen) which is a receiver in the side of muscle of neurotransmitter acetylcholine whereby nervous/muscular transmittance by acetylcholine is inhibited, the protein (X) can be a nicotinic acetylcholine receptor which is the autoantigen. Similarly, in the case of prevention and treatment of hemolytic anemia of autoimmune type, the protein (X) can be an erythrocyte surface marker; in the case of prevention and treatment of idiopathic thrombocytopenic purpura, the protein (X) can be a platelet surface marker; in the case of prevention and treatment of neutropenia of autoimmune type, the protein (X) can be a neutrophil surface marker; in the case of prevention and treatment of hyperthyroidism caused by anti-TSH antibody, the protein (X) can be TSH; in the case of prevention and treatment of primary hypothyroidism (Hashimoto disease), the protein (X) can be a thyroid surface marker; and in the case of prevention of treatment of encephalitis and encephalopathy of autoantibody type, the protein (X) can be NMDA receptor, AMPA receptor, etc.

The protein (X) is not necessary to be the whole receptor or the whole marker but may be a part thereof as far as a recognition site of autoantibodies is contained therein. For example, in the case of myasthenia gravis, the above nicotinic acetylcholine receptor is a multimeric protein consisting of several subunits and, among them, the recognition site of the autoantibodies exists in the N-terminal extracellular region of isoform 1 (an isoform expressed only in skeletal muscle and shown by SEQ ID No: 4) and isoform 2 (an isoform expressed in skeletal muscle, brain, heart, kidney and lung, and shown by SEQ ID No: 5) of an α1 subunit. Accordingly, in the case of myasthenia gravis, the protein (X) may be a nicotinic acetylcholine receptor α1 (nAChRα1) subunit or a part thereof. To be more specific, it may be isoform 1 and/or isoform 2 of nAChRα1 subunit or a part thereof and, to be still more specific, it may consists of amino acid sequence of N-terminal extracellular region of isoform 1 and/or isoform 2 of nAChRα1 subunit.

In the amino acid sequences as such, one or several (such as 1 to 20, preferably 1 to 10, and more preferably 1 to 7) amino acid(s) may be deleted, added and/or substituted as far as the homology thereof is not impaired. With regard to the range thereof, an example is amino acid sequence having 70% or more, preferably 80% or more, and more preferably 90% or more sequence identity. Homology of the amino acid sequence can be calculated using a homology calculation algorithm NCBI BLAST (National Center for Biotechnology Information Basic Local Alignment Search Tool) under such a condition that expectation value is 10, gap is allowed, matrix is BLOSUM 62 and filtering is off. To be more specific, the amino acid sequence where deletion, addition and/or substitution as such are/is introduced can be easily prepared by substituting the corresponding DNA sequence using a commercially available kit such as Site-Directed Mutagenesis Kit (manufactured by Takara Bio Inc.) or QuickChange Site-Directed Mutagenesis Kit (manufactured by STRATAGENE).

The protein (A) in the fusion protein of the present invention is a protein containing a fragment of an antibody heavy chain constant region and it may be, for example, a Fc region of the antibody heavy chain, antibody heavy chain CH1 region or a part of it. With regard to the Fc region of the antibody heavy chain, the amino acid sequence of SEQ ID No: 1 or SEQ ID No: 2 may be specifically exemplified. Both SEQ ID No: 1 and No: 2 are the sequences of human Fc region. SEQ ID No: 1 is a type abundantly found in Asian people while SEQ ID No: 2 is a type abundantly found in European and American people.

A specific example of the fusion protein of the present invention is a protein consisting of amino acid sequence of SEQ ID No: 3. This fusion protein corresponds to the case where the protein (X) is amino acid sequence of N-terminal extracellular region of isoform 1 of nAChRα1 subunit (an amino acid sequence consisting of amino acids of the 1st to 210th positions in SEQ ID No: 4) and the protein (A) is amino acid sequence of SEQ ID No: 1. In this amino acid sequence, as mentioned above, one or several (such as 1 to 20, preferably 1 to 10, and more preferably 1 to 7) amino acid (s) may be deleted, added and/or substituted as far as the homology thereof is not impaired.

The fusion protein of the present invention can be manufactured according to the conventional publicly-known gene engineering technique. Thus, for example, each of DNA coding for the protein (X) and DNA coding for the protein (A) is amplified if necessary, those DNAs are bound each other, the resulting DNA is inserted into an expression vector and a host cell is transfected with the vector to express the fusion protein whereby fusion protein of the present invention can be manufactured. Amplification of DNA can be conducted by, for example, a PCR method and binding of the amplified DNA can be conducted, for example, by an overlap extension PCR method. It is preferred that the expression vector comprises a promoter such as CMV or SV40 for enhancing the expression efficiency and a secretion signal sequence such as antibody heavy chain signal sequence, transmembrane protein signal sequence or antibody K chain signal sequence for making the recovery of the expressed fusion protein easy. As to the host cell, yeast, animal cell, insect cell, plant cell, etc. can be used. Among them, animal cell is preferred and CHO cell, HEK293 cell, etc. are particularly preferred. The expressed fusion protein may be recovered by conventional means and may be purified by, for example, means of a protein A column method.

Now the autoantibody production inhibitor of the present invention characterized by comprising the fusion protein of the present invention as an effective ingredient will be illustrated. Examples of the specific preparation form of such a drug are injection and suppository. In the case of injection, a stabilizer such as saccharide, polyol, albumin or surfactant, an isotonization agent such as salt, etc. are added to the above-prepared fusion protein of the present invention, the resulting product is freeze-dried for preservation and administered by dissolving in water for injection upon use. Although there is no particular limitation for the content of the fusion protein of the present invention in the freeze-dried product, it is 0.01 to 200 mg/g and preferably 0.1 to 100 mg/g, for example. Although there is no particular limitation for the content of the fusion protein in the dissolved injection, it is 0.01 to 200 mg/mL and preferably 0.1 to 100 mg/mL, for example. Examples of the administering method in the case of injection include intravenous administration, intramuscular administration and subcutaneous administration. In the case of suppository, the fusion protein of the present invention may be mixed, for example, with the common base for suppositories followed by administering the resulting preparation to the rectum. Although there is no particular limitation for the content of the fusion protein of the present invention in the suppository, it is 0.1 to 300 mg/mL and preferably 0.5 to 100 mg/mL, for example. Dose of the autoantibody production inhibitor of the present invention varies depending upon the aimed therapeutic effect, administering method, therapeutic period, age, body weight, etc. and it is usually 10 µg/kg to 50 mg/kg per day for an adult.

When the autoantibody production inhibitor of the present invention is administered to a patient of autoimmune disease of autoantibody type, the fusion protein of the present invention in the autoantibody production inhibitor is distributed into the body by flowing on the body fluid flow. When the fusion protein encounters the autoantibodies, the autoantibodies bind to a protein (X) part in the fusion protein (the site recognized by the autoantibodies) and, therefore, these autoantibodies can no longer react with the autoantigen of the patient himself. Further, when the fusion protein of the present invention encounters the B cells which produce the autoantibodies, the fusion protein is bound to the antibodies (B cell receptor) on the surface of these B cells. When such a bond is generated, effector cells such as NK cells bind to a protein (A) part in the fusion protein (a fragment of the antibody heavy chain constant region), exhibit an antibody-dependent cellular cytotoxicity (ADCC activity) and attack the B cells binding to the fusion protein to kill them. As such, in accordance with the present invention, the outcome is not only that the autoantibodies existing in the body are neutralized to prevent the binding of the autoantibodies to the autoantigen but also that the specific B cells which are a production source of the autoantibodies can be selectively killed. Accordingly, the autoantibody production inhibitor of the present invention is expected to exhibit an effect as a preventive/treating drug for autoimmune disease of autoantibody type such as myasthenia gravis.

### Examples

The present invention will now be illustrated in more detail as hereunder by Examples, but the present invention is not limited to these Examples.

### (1) Manufacture of fusion protein

Gene of human antibody heavy chain signal sequence (secretion signal sequence) was amplified using a gene amplification kit "KOD-plus-Ver.2" (catalog number: KOD-211) of Toyobo where cDNA of human peripheral blood lymphocytes (PBL) was used as a template and DNA sequences of SEQ ID No: 8 and SEQ ID No: 9 were used as primers.

The amplified gene was subjected to cloning using a cloning kit "Zero Blunt TOPO PCR Cloning Kit for Sequence" (catalog number: K2895-20) of Invitrogen and analysis of the gene sequence was conducted. After that, an expression vector "pcDNA3.1(+)Vector" (catalog number: V790-20) of Invitrogen was treated with restriction enzymes NheI (catalog number: R0131) and EcoRI (catalog number: R0101) of NEB and the amplified antibody heavy chain signal sequence (secretion signal sequence) was inserted into the expression vector.

Then the gene in N-terminal extracellular region of isoform 1 of human nAChRα1 subunit was amplified using "KOD-plus-Ver.2" of Toyobo where cDNA of human nAChRα1 subunit (catalog number: MHS1011-61598) purchased from Open Biosystems was used as a template and DNA sequences of SEQ ID No: 10 and SEQ ID No: 11 were used as primers. The amplified gene corresponds to the 1st to 210th positions in the amino acid sequence of SEQ ID No: 4. This amino acid sequence is shown in SEQ ID No: 6.

On the other hand, gene of human antibody heavy chain Fc region was amplified by PCR using cDNA of human peripheral blood lymphocyte (PBL) as a template and using DNA sequences of SEQ ID No: 12 and SEQ ID No: 13 as primers. The amplified gene was subjected to cloning using "Zero Blunt TOPO Cloning Kit for Sequence" of Invitrogen and analysis of the gene sequence was conducted. As a result, it was found that genes of two types of human antibody heavy chain Fc regions coding for amino acid sequences of SEQ ID No: 1 and SEQ ID No: 2 were obtained.

The gene of N-terminal extracellular region of isoform 1 of human nAChRα1 subunit prepared as such and the gene of human antibody heavy chain Fc region were mixed and the genes were amplified by an overlap extension PCR method using DNA sequences of SEQ ID No: 10 and SEQ ID No: 13 as primers to prepare a gene sequence which codes for N-terminal extracellular region of isoform 1 of human nAChRα1 subunit and for human antibody heavy chain Fc region in a single chain. The amino acid sequences corresponding to the prepared gene sequences are shown in SEQ ID No: 3 and NO: 7. SEQ ID No: 3 corresponds to the case where the human antibody heavy chain Fc region is amino acid of SEQ ID No: 1 while SEQ ID No: 7 corresponds to the case where the human antibody heavy chain Fc region is amino acid of SEQ ID No: 2.

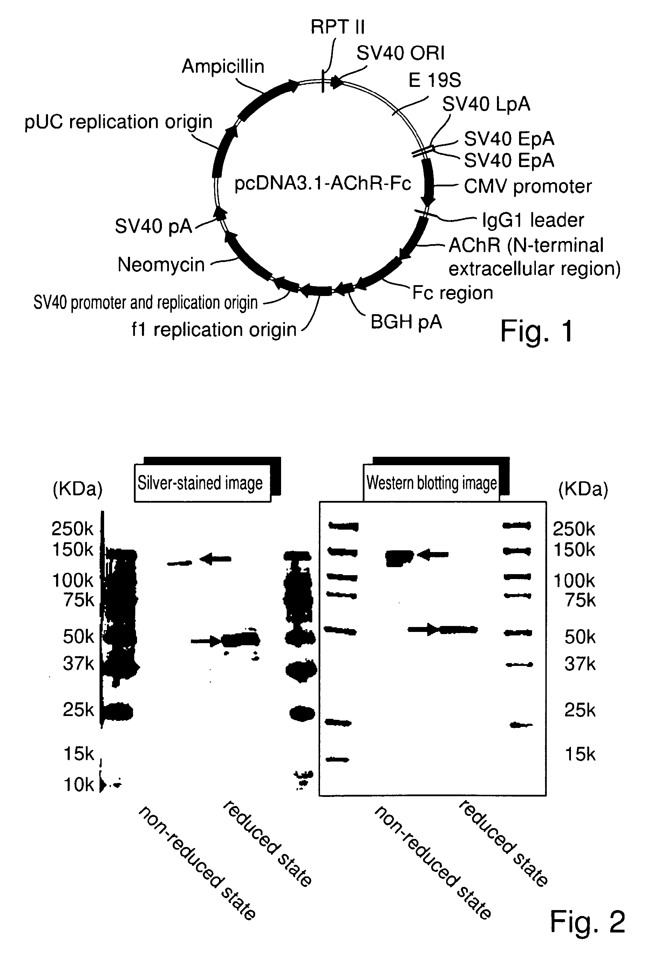

The prepared gene sequence was cloned using "Zero Blunt TOPO Cloning Kit for Sequence" of Invitrogen and analysis of the gene sequence was conducted. After that, pcDNA 3.1 vector into which secretion signal was previously inserted was treated with restriction enzymes PpuMI (catalog number: R0506) and PmeI (catalog number: R0560) of NEB and the prepared gene sequence was inserted into the treated vector to prepare a vector for expressing a fusion protein (α1-Fc) consisting of an N-terminal extracellular region of isoform 1 of human nAChRα1 subunit and a human antibody heavy chain Fc region. A schematic chart of the resulting vector is shown in Fig. 1.

After that, HEK 293 cells were transfected with the resulting vector using an expression system "Free Style MAX 293 Expression System" (catalog number: K9000-10) of Invitrogen to express the fusion protein α1-Fc. Then it was purified using a purifying column "HiTrap Protein A HP Column" (catalog number: 17-0402-01) of GE Health Care to give the fusion protein α1-Fc.

Confirmation of expression of the fusion protein was conducted by means of a silver staining and a western blotting after an SDS-PAGE. For the western blotting, an HRP-labeled anti-human IgG antibody was used. The result is shown in Fig. 2. A band shown by an arrow in Fig. 2 corresponds to the fusion protein α1-Fc.

(2) Confirmation of reactivity of fusion protein to autoantibody
A hybridoma Mab35 (ATCC number: TIB-175) which produces an anti-nAChR autoantibody mAb35 (an autoantibody to rat nAChR) was incubated and, from its supernatant fluid, an anti-nAChR autoantibody mAb35 was purified using a purifying column "HiTrap Protein G HP column" (catalog number: 17-0404-01) of GE Health Care. It has been reported by Tzartos, et al. that this antibody shows a cross reaction not only to rats but also to mice and humans (J. Neuroimmunol,1987; 15, 185-94).

After that, the purified anti-nAChR autoantibody was immobilized on an ELISA plate under various concentrations to conduct a blocking. Then it was made to react with the fusion protein α1-Fc prepared in (1), treated with an HRP-labeled anti-human Fc antibody attached to an ELISA quantitation kit "IgG (Fc), Human, ELISA Quantitation Kit" (catalog number: E80-104) of Funakoshi and made to react with a substrate solution "TMB Solution" (catalog number: N301) of Funakoshi as a substrate and the reaction was stopped by 1% sulfuric acid. After that, absorbance at 450 nm wavelength was measured.

The result is shown in the following Table 1. As will be apparent from Table 1, the more the concentration of the autoantibody, the more the absorbance. Thus, it was confirmed that the fusion protein reacts with the autoantibody depending upon the concentration of the autoantibody.

[Table 1]

**Table 1 Confirmation of reactivity of fusion protein to autoantibody**

| Concentration of the autoantibody (ng/mL) | Absorbance A450nm |
|---|---|
| 10000 | 1.233 |
| 2500 | 1.120 |
| 625 | 0.871 |
| 156.3 | 0.659 |
| 39.1 | 0.557 |
| 9.8 | 0.505 |

### (3) Confirmation of binding of fusion protein α1-Fc to hybridoma Mab35

In a living body, autoantibodies are produced in B cells. On the cell membrane of the cell surface of the B cells producing the autoantibodies, the same antibodies as the autoantibodies are presented as a B cell receptor. Since the hybridoma Mab35 used in (2) produces an anti-nAChR autoantibody, it is likely that the hybridoma Mab35 also presents the autoantibody on the cell surface the same as in the case of B cell. Under such circumstances, it was now confirmed whether the fusion protein α1-Fc will bind to the hybridoma Mab35.

Various concentrations of the fusion protein α1-Fc prepared in (1) was added to 1 × 10⁵ cells of the hybridoma Mab35 washed with HBSS/BSA. Then a PE-labeled anti-human IgG antibody (catalog number: 109-116-170) of Jackson Immuno Research Laboratories was added thereto followed by washing with HBSS/BSA and the bond between the fusion protein and the hybridoma was measured by a flow cytometer.

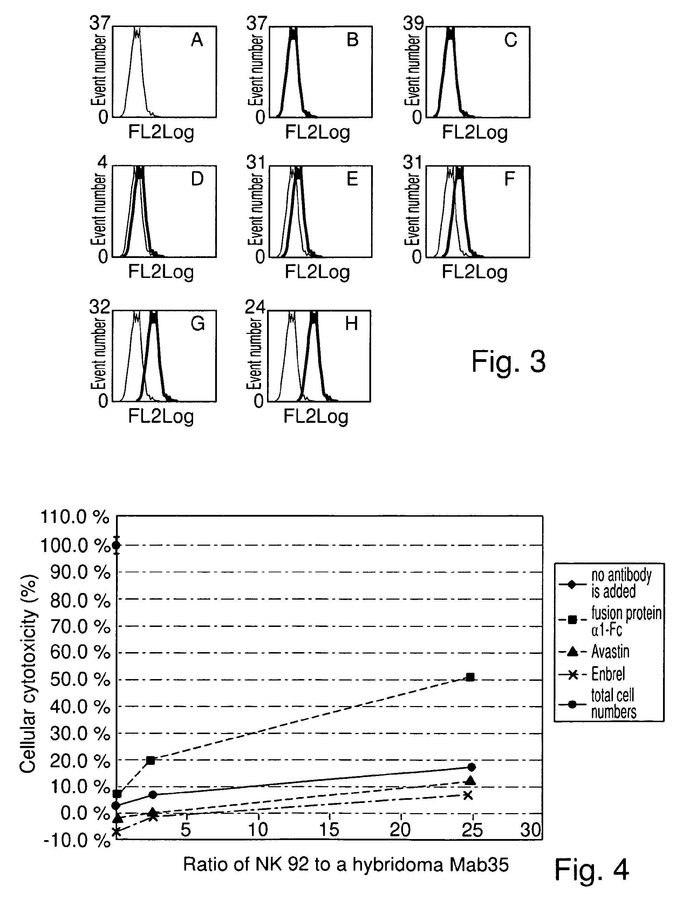

The result is shown in Fig. 3. It is apparent from Fig. 3 that the fusion protein α1-Fc binds to the hybridoma Mab35 in a concentration-dependent manner. Such a result shows the possibility that the fusion protein α1-Fc binds to anti-nAChR autoantibody-producing B cells in the body of a patient suffering from myasthenia gravis.

### (4) Confirmation of ADCC activity of fusion protein α1-Fc to hybridoma Mab35

Hybridoma Mab35 (1 × 10⁶ cells) was washed with HBSS/BSA and incubated at 37°C for 30 minutes in HBSS/BSA to which a fluorescent dye "Calcein-AM" (catalog number: C326) of Dojinsha was added to make the concentration 10 µM whereby Calcein-AM was incorporated into the hybridoma cells. After that, those hybridoma cells were disposed to a 96-well plate to make 10000 cells per well and then the fusion protein α1-Fc prepared in (1) or a control antibody (Avastin or Enbrel) and NK 92 cells (effector cells, ATCC number: CRL-2407) were added thereto in various concentrations followed by incubating at 37°C for 4 hours. After the incubation, centrifugal separation was conducted at ×300 g for 5 minutes to precipitate the cells and fluorescence of each supernatant liquid was measured (Ex=485nm, Em=540nm).

The result is shown in Fig. 4. As will be apparent from Fig. 4, although a very weak cellular cytotoxicity (natural killing) was noted in a dependently manner on NK92 cell numbers even in a group to which only NK 92 cells were added (no antibody, etc. added), a strong cellular cytotoxicity of 50% at the highest was noted in a dependently manner on NK 92 cell numbers in a group to which the fusion protein α1-Fc was administered. On the other hand, no strong cellular cytotoxicity was noted in a group to which the control antibody was administered.

It is apparent from the above result that the fusion protein α1-Fc binds to the hybridoma Mab35 and exhibits the cellular cytotoxicity (ADCC activity) by the NK 92 cells (effector cells) via a protein (A) part (Fcγ receptor) in the fusion protein α1-Fc. This result shows the possibility that, in the body of a patient suffering from myasthenia gravis, the fusion protein α1-Fc binds to the anti-nAChR autoantibody-producing B cells to attack and kill the autoantibody-producing B cells due to the ADCC activity.

### Industrial Applicability

The autoantibody production inhibitor of the present invention can prevent or treat the autoimmune disease of autoantibody type by two ways which are inhibition of the autoantibody production and neutralization of the produced autoantibodies. Therefore, the autoantibody production inhibitor of the present invention can be widely used for effectively preventing and treating various autoimmune diseases of autoantibody type such as myasthenia gravis. Sequence Listing Free Text

Sequence ID Nos. 8-13 are the sequences of the primers.

## Claims

1. An autoantibody production inhibitor comprising, as an effective ingredient, a fusion protein which consists of a protein (X) containing a site recognized by autoantibodies which are a cause of the autoimmune disease of autoantibody type and a protein (A) containing a fragment which exhibits the antibody-dependent cellular cytotoxicity of the antibody heavy chain constant region.

2. The autoantibody production inhibitor according to claim 1, wherein the protein (A) contains a Fc region of the antibody heavy chain, antibody heavy chain CH1 region or a part thereof.

3. The autoantibody production inhibitor according to claim 1, wherein the protein (A) consists of amino acid sequence of SEQ ID No: 1 or SEQ ID No: 2.

4. The autoantibody production inhibitor according to claim 1, wherein the protein (X) is a nicotinic acetylcholine receptor α1 (nAChRα1) subunit or a part thereof.

5. The autoantibody production inhibitor according to claim 4, wherein the protein (X) is isoform 1 and/or isoform 2 of nAChRα1 subunit or a part thereof.

6. The autoantibody production inhibitor according to claim 5, wherein the isoform 1 consists of amino acid sequence of SEQ ID No: 4.

7. The autoantibody production inhibitor according to claim 5, wherein the isoform 2 consists of amino acid sequence of SEQ ID No: 5.

8. The autoantibody production inhibitor according to claim 5, wherein the protein (X) consists of amino acid sequence of N-terminal extracellular region of isoform 1 and/or isoform 2 of nAChRα1 subunit, or consists of amino acid sequence wherein one or several amino acid(s) is/are deleted, added and/or substituted in said amino acid sequence.

9. The autoantibody production inhibitor according to claim 4, wherein the fusion protein consists of amino acid sequence of SEQ ID No: 3, or consists of amino acid sequence wherein one or several amino acid(s) is/are deleted, added and/or substituted in the amino acid sequence of SEQ ID No: 3.

10. The autoantibody production inhibitor according to claim 1, wherein the autoantibody production inhibitor is in a form of injection and suppository.
